# EUROPÄISCHE PATENTSCHRIFT

(11) **EP 2 380 003 B1**
(45) Veröffentlichungstag und Bekanntmachung des Hinweises auf die Patenterteilung: **25.07.2012**
(21) Anmeldenummer: 10701116.5
(22) Anmeldetag: 12.01.2010
(51) Int. Cl.: G01N 21/05, G01N 21/77, A61B 5/00, A61B 5/1455

(54) **MESSANORDNUNG ZUR BESTIMMUNG ZUMINDEST EINES PARAMETERS EINER BLUTPROBE**
MEASURING ARRANGEMENT FOR DETERMINING AT LEAST ONE PARAMETER OF A BLOOD SAMPLE
DISPOSITIF DE MESURE POUR DÉTERMINER AU MOINS UN PARAMÈTRE D'UN ÉCHANTILLON SANGUIN

(30) Priorität: 19.01.2009 AT 792009
(43) Veröffentlichungstag der Anmeldung: 26.10.2011
(73) Patentinhaber: Smart Medical Solutions GmbH, 8047 Graz (AT)
(72) Erfinder: KÖHLER, Hans, A-8046 Graz (AT)
(74) Vertreter: Babeluk, Michael
(86) Internationale Anmeldenummer: PCT/EP2010/050239
(87) Internationale Veröffentlichungsnummer: WO 2010/081790

(56) Entgegenhaltungen:
- EP-A1- 0 793 090
- EP-A2- 0 175 352
- EP-A2- 1 106 987
- US-A1- 2003 190 262

## Beschreibung

Die Erfindung betrifft eine Messanordnung zur Bestimmung zumindest eines Parameters einer Blutprobe, mit einer Durchflussmesszelle, in der zumindest ein mit der Blutprobe in Kontakt bringbares lumineszenzoptisches Sensorelement angeordnet ist, mit zumindest einer Lichtquelle zur Anregung des lumineszenzoptischen Sensorelementes und zumindest einem Fotodetektor zur Aufnahme der vom lumineszenzoptischen Sensorelement emittierten Lumineszenzstrahlung, wobei die Lichtquelle und der Fotodetektor an gegenüberliegenden Seiten und der Durchflussmesszelle angeordnet sind.

Aus der EP 0 175 352 B1 ist ein Verfahren und eine Anordnung zur schnellen Messung der Parameter eines Probenmediums bekannt geworden. Die Anordnung weist eine Durchflussmesszelle für Gase und Flüssigkeiten auf, mit der die gleichzeitige Bestimmung mehrerer Parameter möglich ist, wobei eine transparente, lumineszierende Sensorschicht in der Durchflusszelle mit der Probe in Kontakt steht. Als Messgrößen werden die Sauerstoffkonzentration und die Temperatur genannt, wobei die gemessene Lumineszenzstrahlung bei 650 nm bzw. 720 nm liegt und die Anregungsstrahlung eine kürzere Wellenlänge aufweist. Die Strahlungsanregung erfolgt über LEDs und die Strahlungsdetektion über Fotodioden, die sich auf gegenüberliegenden Seiten der Durchflussmesszelle befinden. Die lumineszierende Sensorschicht ist detektorseitig angeordnet, so dass die Anregungsstrahlung auf ihrem Weg zur lumineszierenden Sensorschicht das Probenmedium durchdringen muss. Diese Tatsache stellt sich bei absorbierenden Flüssigkeiten wie Blut als Nachteil heraus, da die Anregungsstrahlung durch das Messmedium erheblich abgeschwächt würde.

Weiters ist aus der EP 1 130 382 B1 ein optischer Sensor zum Nachweis mehrerer Analyte einer Fluidprobe bekannt, bei welchem eine Mehrzahl von optischen Sensoren mit der Fluidprobe in Kontakt steht. Die Vorrichtung weist Lichtquellen zum Bereitstellen einer Anregungsstrahlung und Detektoren zur Bestimmung der Lichtinteraktion durch die Sensoren auf, wobei ein Prozessor vorhanden ist, der aus den gemessenen Lichtinteraktionen die Konzentration jedes der Analyten in der Fluidprobe bestimmt. Der Sensor dient unter anderem zur Messung von Glucose im Blut, wobei auch die O₂- Konzentration und die Temperatur bestimmt werden.

Es sind auch Messanordnungen bekannt (EP 1 106 987 B1), bei welchen die beiden optischen Komponenten (Lichtquelle und Detektor) die Sensorschicht in der Durchflussmesszelle von einer Seite kontaktieren, wobei Teile der Messzelle transparent sind und als Lichtleiter für die Messstrahlung und die Anregungsstrahlung verwendet werden.

Aus der WO 2002/059585 A2 ist eine Messanordnung zur Bestimmung der Sauerstoffkonzentration eines Gases, beispielsweise der Atemluft, bekannt. Die Messanordnung weist eine Durchflussmesszelle auf, in welcher ein den Gasstrom kontaktierendes, lumineszenzoptisches Sensorelement angeordnet ist. Als mögliche Messgeometrien werden zwei Reflexionsgeometrien dargelegt, bei welchen sich die Strahlungsquelle für die Anregungsstrahlung und der Detektor zur Aufnahme der Lumineszenzstrahlung auf der selben Seite der Durchflussmesszelle befinden. Weiter wird eine Durchlichtgeometrie beschrieben, bei welcher das lumineszenzoptische Sensorelement an der Detektorseite der Durchflussmesszelle angeordnet ist.

Aufgabe der Erfindung ist, bei einer Messanordnung zur Bestimmung zumindest eines Parameters einer Blutprobe Verbesserungen der Signalgüte der Messstrahlung zu erzielen, wobei die Messanordnung kostengünstig und einfach herstellbar sein soll.

Diese Aufgabe wird erfindungsgemäß dadurch gelöst, dass das zumindest eine lumineszenzoptische Sensorelement an der der Lichtquelle zugekehrten Anregungsseite der Durchflussmesszelle angeordnet ist, dass die Lichtquelle eine Anregungsstrahlung kleiner 600 nm, beispielsweise von 425 nm, emittiert und die Lumineszenzstrahlung der lumineszenzoptischen Sensorelemente in einem Wellenlängenbereich größer 600 nm liegt, wodurch die Anregungsstrahlung durch die Blutprobe einer weit stärkeren Absorption ausgesetzt ist als die Lumineszenzstrahlung.

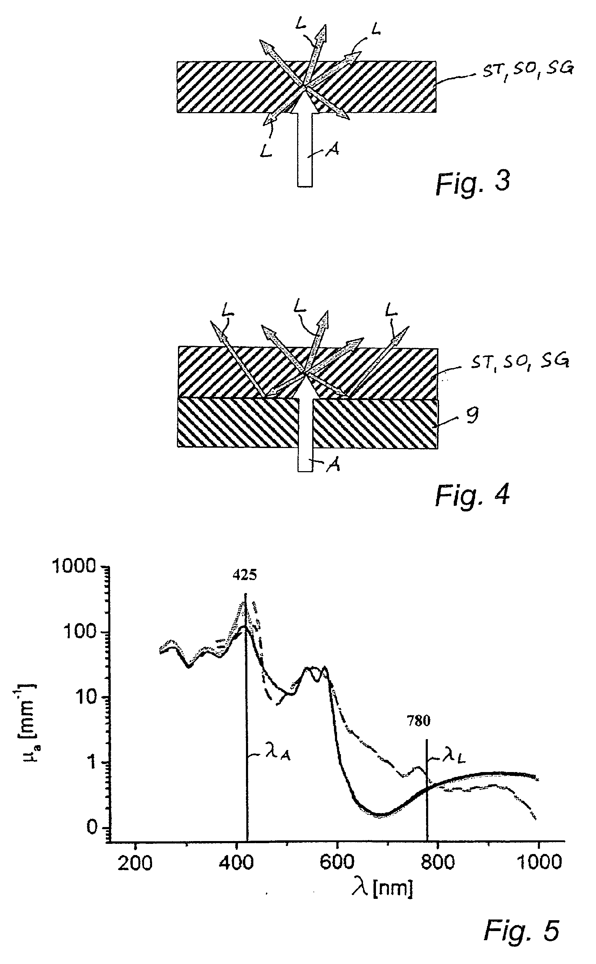

Die Erfindung nützt die Tatsache aus, dass Blut eine sehr große Wellenlängen-Abhängigkeit bei der Strahlungsabsorption aufweist, welche beispielsweise in der Abbildung gemäß Fig. 5 dargestellt ist. In dieser Darstellung werden sowohl für oxigeniertes Blut (durchgezogene Linie) als auch für desoxigeniertes Blut (strichlierte Linie) die Absorption µₐ in Abhängigkeit von der Wellenlänge λ dargestellt. Das Diagramm stammt aus Faber et al: "Oxygen Saturation-Dependent Absorption and Scattering of Blood"; Physical Review Letters, 2004 und behandelt Unterschiede im Absorptionsverhalten von oxigeniertem und desoxigeniertem Blut. Aus diesem Diagramm ist ersichtlich, dass beispielsweise eine Anregungsstrahlung λ_{A} im Bereich von 425 nm im Blut weit stärker absorbiert wird, als die von den optischen Sensoren emittierte Lumineszenzstrahlung λ_{L} im Bereich von 780 nm. Im dargestellten Beispiel wird das Anregungslicht ca. um den Faktor 100 gegenüber dem Lumineszenzlicht abgeschwächt. Durch die erfindungsgemäße Anordnung der lumineszenzoptischen Sensorelemente auf der Anregungsseite der Durchflussmesszelle kommt es dazu, dass die Blutprobe als Filter für die Anregungsstrahlung dient und bevorzugt die von den Sensorelementen emittierte Lumineszenzstrahlung zu den Fotodetektoren gelangt.

Erfindungsgemäß sind die optischen Sensorelemente bevorzugt in linearer Anordnung längs der Messzellenachse an der Anregungsseite der Durchflussmesszelle angeordnet, wobei jedem Sensorelement eine Lichtquelle und auf der gegenüberliegenden Messseite der Durchflussmesszelle ein Fotodetektor zugeordnet ist.

Ein weiterer Vorteil im Vergleich zum Stand der Technik, beispielsweise der EP 0 175 352 B1, besteht darin, dass die Durchflussmesszelle auswechselbar in eine zweiteilige Messmanschette einsetzbar ist, deren Anregungsteil die Lichtquellen, vorzugsweise LEDs, samt Anregungselektronik und deren Messteil die Fotodetektoren, vorzugsweise Fotodioden, samt Messelektronik enthält. Die Lichtquellen und Detektoren befinden sich dadurch auf räumlich getrennten Schaltkreisen, wodurch die elektronische Beeinflussung der einzelnen Komponenten vermieden wird. Die Blutprobe ist dabei nur für Wellenlängen größer 600 nm ausreichend durchlässig, die Anregungsstrahlung liegt bei kürzeren Wellenlängen, beispielsweise bei 425 nm.

Gemäß einer vorteilhaften Ausgestaltung der Erfindung weist die Anregungsseite der Durchflussmesszelle im Bereich der lumineszenzoptischen Sensorelemente eine Spiegelschicht auf, die für die Anregungsstrahlung durchlässig ist und die Lumineszenzstrahlung reflektiert. Mit Hilfe der Spiegelschicht werden die in Richtung Lichtquelle emittierten Strahlungsanteile der Lumineszenzstrahlung zum Fotodetektor umgeleitet und das Nettosignal dadurch verstärkt.

Für den Fall einer Phasenmessung ist an der Anregungsseite der Messzelle zumindest eine Referenzlichtquelle angeordnet, deren Referenzstrahlung die Durchflussmesszelle durchsetzt und in die auf der gegenüberliegenden Messseite angeordneten Fotodetektoren gelangt.

Die Erfindung wird im Folgenden anhand von Zeichnungen näher erläutert. Es zeigen:
- Fig. 1: eine erste Ausführungsvariante einer Messanordnung zur Bestimmung zumindest eines Parameters einer Blutprobe in einem schematischen Längsschnitt;
- Fig. 2: eine zweite Ausführungsvariante der Messanordnung in einer Schnittdarstellung gemäß Fig. 1;
- Fig. 3: und Fig. 4 Detaildarstellungen aus den Messanordnungen gemäß Fig. 1 und Fig. 2; sowie
- Fig. 5: das Absorptionsdiagramm von Blut im Bereich einer Wellenlänge λ von 200 nm bis 1.000 nm. durchgezongene Linien : oxigeniertes Blut
- strichlierte Linien : desoxigeniertes Blut

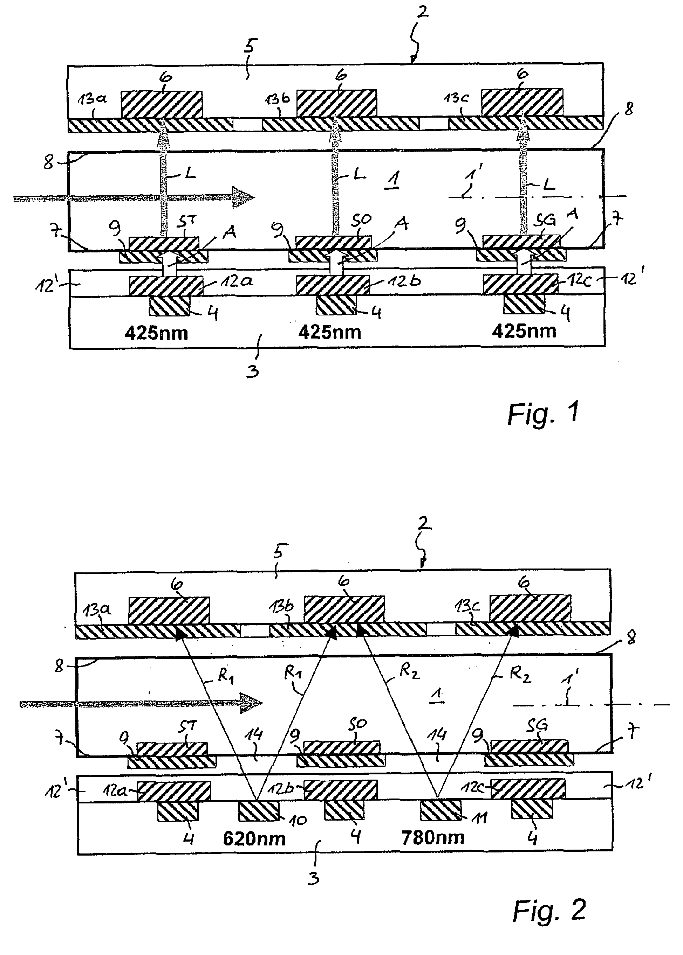

Die in Fig. 1 dargestellte Messanordnung zur Bestimmung zumindest eines Parameters einer Blutprobe weist eine Durchflussmesszelle 1 auf, in der beispielsweise drei lumineszenzoptische Sensorelemente ST (Temperatur), SO (Sauerstoff) und SG (Glucose) angeordnet sind, die bei der Messung mit der Blutprobe in Kontakt gebracht werden. Die Durchflussmesszelle 1 ist auswechselbar in einer zweiteiligen Messmanschette 2 angeordnet (eingesteckt bzw. eingeklickt), deren Anregungsteil 3 die den einzelnen Sensorelementen zugeordneten Lichtquellen 4 und Anregungsfilter 12a bis 12c samt der nicht weiter dargestellten Anregungselektronik enthält, wobei der Messteil 5 der Messmanschette 2 die Fotodetektoren 6 und Messfilter 13a bis 13c samt Messelektronik (nicht dargestellt) enthält. Die Lichtquelle 4 und die Fotodetektoren 6 sind an gegenüberliegenden Seiten 7, 8 (Anregungsseite 7 und Messseite) der Durchflussmesszelle 1 angeordnet.

Da die lumineszenzoptischen Sensorelemente ST, SO und SG an der der Lichtquelle 4 zugekehrten Anregungsseite 7 der Durchflussmesszelle 1 angeordnet sind, durchdringt sowohl die im lumineszenzoptischen Sensorelement entstehende Lumineszenzstrahlung L als auch ein Teile der Anregungsstrahlung A die Blutprobe. Die Anregungsstrahlung A wird in einem weit größeren Ausmaß durch Absorption in der Blutprobe geschwächt als die langwelligere Lumineszenzstrahlung, so dass durch die Probe ein für die Messung positiver Filtereffekt entsteht der die Signalgüte verbessert.

Die lumineszenzoptischen Sensorelemente ST, SO, SG sind bevorzugt in einer linearen Anordnung längs der Messzellenachse 1' angeordnet, wobei jedem Sensorelement eine Lichtquelle 4 und auf der gegenüberliegenden Messseite 8 der Durchflussmesszelle 1 ein Fotodetektor 6 zugeordnet ist.

Mit der in Fig. 1 skizzierten Messanordnung kann beispielsweise eine Abklingzeitmessung durchgeführt werden, d.h. die nach Anregung der lumineszenzoptischen Sensorelemente gemessene Abklingzeit der Lumineszenzintensität ist ein Maß für die Messgröße.

Wie beispielsweise in Fig. 1 dargestellt, kann die Anregungsseite 7 der Durchflussmesszelle 1 im Bereich der lumineszenzoptischen Sensorelemente ST, SO, SG eine Spiegelschicht 9 aufweisen, wobei die Wirkung der Spiegelschicht anhand der Figuren 3 und 4 dargestellt ist. Die Anregungsstrahlung A trifft auf das lumineszenzoptische Sensorelement ST, SO oder SG, wobei Lumineszenzstrahlung L in alle Raumrichtungen freigesetzt wird. Ohne Spiegelschicht (siehe Fig. 3) bringen jene Strahlungsanteile, die in Richtung Lichtquelle emittiert werden keinen Beitrag zum Messsignal.

Bei der Anbringung einer Spiegelschicht (s. Fig. 4), welche für Strahlung einer Wellenlänge kleiner 600 nm durchlässig ist und Strahlung einer Wellenlänge größer 600 nm reflektiert, werden zusätzlich Anteile der Lumineszenzstrahlung L in den Detektor reflektiert und verstärken das Messsignal.

Mit der in Fig. 2 skizzierten Messanordnung kann beispielsweise eine Phasenmessung durchgeführt werden, bei welcher für die Referenzierung der Messsignale ein Referenzsignal gewonnen werden muss. Erfindungsgemäß ist an der Anregungsseite 7 der Durchflussmesszelle 1 zumindest eine Referenzlichtquelle 10, und/oder 11 angeordnet, deren Referenzstrahlung R₁, R₂ die Durchflussmesszelle 1 durchsetzt und durch die auf der gegenüberliegenden Messseite 8 angeordneten Fotodetektoren 6 detektiert wird. Beispielsweise kann eine erste Referenzlichtquelle 10 mit einer Referenzstrahlung R₁ im Bereich vom 620 nm und eine zweite Referenzlichtquelle 11 mit einer Referenzstrahlung R₂ im Bereich von 780 nm vorgesehen sein, welche vorzugsweise im Anregungsteil 3 der zweiteiligen Messmanschette 2 angeordnet sind. In der Spiegelschicht 9 an der Anregungsseite 7 der Durchflussmesszelle 1 sind Öffnungen 14 vorgesehen, durch die die Referenzstrahlung in die Durchflussmesszelle 1 eintreten kann.

Sowohl in Fig. 1 als auch in Fig. 2 sind zwischen den Lichtquellen 4 und den lumineszenzoptischen Sensorelementen ST, SO, SG Anregungsfilter 12a, 12b, und 12c angeordnet, wobei die Lichtquellen 4 auch in eine gemeinsame Filterschicht 12' eingebettet sein können. Weiters sind an der Eintrittsseite der Fotodetektoren 6 Messfilter 13a, 13b und 13c angeordnet.

Die LEDs der Lichtquellen 4 können z.B. eine Anregungsstrahlung kleiner 600 nm, beispielsweise von 425 nm, emittieren, wobei die Lumineszenzstrahlung der lumineszenzoptischen Sensorelemente ST, SO, SG in einem Wellenlängenbereich größer 600 nm z.B. bei 780 nm liegt.

Zusammengefasst bestehen die Vorteile der erfindungsgemäßen Messvorrichtung darin:
- sehr einfacher optischer Aufbau;
- flaches Design der Durchflussmesszelle 1 und der Messmanschette 2;
- große Signalintensität, weil die Fotodetektoren größer ausgeführt werden können;
- kein elektrisches Übersprechen (minimaler Signaluntergrund), weil es zwischen dem Anregungsteil 3 und dem Messteil 5 der Messmanschette 2 eine räumliche Trennung gibt;
- über die Messung der Signalintensität erfolgt die Identifizierung der Probe (Blutprobe oder Spülflüssigkeit);
- simultane fotometrische Charakterisierung der Blutprobe (Hämoglobingehalt und Oxigenierung);
- Parallelbestimmung des arteriellen pO₂.

## Patentansprüche

1. Messanordnung zur Bestimmung zumindest eines Parameters einer Blutprobe, mit einer Durchflussmesszelle (1), in der zumindest ein mit der Blutprobe in Kontakt bringbares lumineszenzoptisches Sensorelement (ST, SO, SG) angeordnet ist, mit zumindest einer Lichtquelle (4) zur Anregung des lumineszenzoptischen Sensorelementes und zumindest einem Fotodetektor (6) zur Aufnahme der vom lumineszenzoptischen Sensorelement emittierten Lumineszenzstrahlung, wobei die Lichtquelle (4) und der Fotodetektor (6) an gegenüberliegenden Seiten (7) und (8) der Durchflussmesszelle (1) angeordnet sind, **dadurch gekennzeichnet, dass** das zumindest eine lumineszenzoptische Sensorelement (ST, SO, SG) an der der Lichtquelle (4) zugekehrten Anregungsseite (7) der Durchflussmesszelle (1) angeordnet ist, dass die Lichtquelle (4) eine Anregungsstrahlung kleiner 600 nm, beispielsweise von 425 nm, emittiert und die Lumineszenzstrahlung der lumineszenzoptischen Sensorelemente (ST, SO, SG) in einem Wellenlängenbereich größer 600 nm liegt, wodurch die Anregungsstrahlung durch die Blutprobe einer weit stärkeren Absorption ausgesetzt ist als die Lumineszenzstrahlung.

2. Messanordnung nach Anspruch 1, **dadurch gekennzeichnet, dass** an der Anregungsseite (7) der Durchflussmesszelle (1) mehrere lumineszenzoptische Sensorelemente (ST, SO, SG), bevorzugt in linearer Anordnung längs der Messzellenachse (1'), vorliegen, wobei jedem Sensorelement (ST, SO, SG) eine Lichtquelle (4) und auf der gegenüberliegenden Messseite (8) der Durchflussmesszelle (1) ein Fotodetektor (6) zugeordnet ist.

3. Messordnung nach Anspruch 1 oder 2, **dadurch gekennzeichnet, dass** die Durchflussmesszelle (1) auswechselbar in einer zweiteiligen Messmanschette (2) einsetzbar ist, deren Anregungsteil (3) die Lichtquellen (4), vorzugsweise LEDs, samt Anregungselektronik und deren Messteil (5) die Fotodetektoren (6), vorzugsweise Fotodioden, samt Messelektronik enthält.

4. Messordnung nach einem der Ansprüche 1 bis 3, **dadurch gekennzeichnet, dass** die Anregungsseite (7) der Durchflussmesszelle (1) im Bereich der lumineszenzoptischen Sensorelemente (ST, SO, SG) eine Spiegelschicht (9) aufweist, die für die Anregungsstrahlung durchlässig ist und die Lumineszenzstrahlung reflektiert.

5. Messordnung nach einem der Ansprüche 1 bis 4, **dadurch gekennzeichnet, dass** an der Anregungsseite (7) der Durchflussmesszelle (1) zumindest eine Referenzlichtquelle (10, 11) angeordnet ist, deren Referenzstrahlung die Durchflussmesszelle (1) durchsetzt und in die auf der gegenüberliegenden Messseite (8) angeordneten Fotodetektoren (6) gelangt.

6. Messordnung nach Anspruch 5, **dadurch gekennzeichnet, dass** eine erste Referenzlichtquelle (10) mit einer Referenzstrahlung im Bereich vom 620 nm und eine zweite Referenzlichtquelle 11 mit einer Referenzstrahlung im Bereich von 780 nm vorgesehen sind, welche vorzugsweise im Anregungsteil (3) der zweiteiligen Messmanschette (2) angeordnet sind.

7. Messordnung nach einem der Ansprüche 1 bis 6, **dadurch gekennzeichnet, dass** zwischen den Lichtquellen (4) und den lumineszenzoptischen Sensorelementen (ST, SO, SG) Anregungsfilter (12a, 12b, 12c) angeordnet sind oder dass die Lichtquellen (4) in eine Filterschicht (12') eingebettet sind.

8. Messordnung nach einem der Ansprüche 1 bis 7, **dadurch gekennzeichnet, dass** an der Eintrittsseite der Fotodetektoren (6) Messfilter (13a, 13b, 13c) angeordnet sind.

9. Messordnung nach einem der Ansprüche 1 bis 8, **dadurch gekennzeichnet, dass** die Durchflussmesszelle (1) zumindest einen O₂-Sensor (SO), einen Glucosesensor (SG) und einen Temperatursensor (ST)aufweist.

## Claims

1. Measuring arrangement for determining at least one parameter of a blood sample, comprising a flow-through measuring cell (1), in which is disposed at least one luminescence-optical sensor element (ST, SO, SG), which can be brought into contact with the blood sample, and at least one light source (4) for excitation of the luminescence-optical sensor element and at least one photodetector (6) for receiving the luminescence radiation emitted by the luminescence-optical sensor element, the light source (4) and the photodetector (6) being located on opposite sides (7) and (8) of the flow-through measuring cell (1) **characterised in that** the at least one luminescence-optical sensor element (ST, SO, SG) is placed on the excitation side (7) of the flow-through measuring cell (1), which faces the light source (4), and that the light source (4) emits excitation radiation of wavelength less than 600 nm, for instance 425 nm, and that the luminescence radiation of the luminescence-optical sensor elements (ST, SO, SG) lies in a wavelength range greater than 600 nm, thus exposing the excitation radiation to much stronger absorption by the blood sample than the luminescence radiation.

2. Measuring arrangement according to claim 1, **characterised in that** a plurality of luminescence-optical sensor elements (ST, SO, SG) are provided on the excitation side (7) of the flow-through measuring cell (1), preferably in linear array along the measuring cell axis (1'), each sensor element (ST, SO, SG) being assigned a light source (4) and, on the opposite measuring side (8) of the flow-through measuring cell (1), a photodetector (6).

3. Measuring arrangement according to claim 1 or 2, **characterised in that** the flow-through measuring cell (1) can exchangeably be inserted into a two-part measuring sleeve (2), whose excitation part (3) contains the light sources (4), preferably LEDs, plus excitation electronics, and whose measuring part (5) contains the photodetectors (6), preferably photodiodes, plus measuring electronics.

4. Measuring arrangement according to any of claims 1 to 3, **characterised in that** the excitation side (7) of the flow-through measuring cell (1) is provided with a mirror layer (9) in the area of the luminescence-optical sensor elements (ST, SO, SG), which layer is transparent for the excitation radiation and reflects the luminescence radiation.

5. Measuring arrangement according to any of claims 1 to 4, **characterised in that** on the excitation side (7) of the flow-through measuring cell (1) at least one reference light source (10, 11) is provided, whose reference radiation passes through the flow-through measuring cell (1) and reaches the photodetectors (6) on the opposite measuring side (8).

6. Measuring arrangement according to claim 5, **characterised in that** a first reference light source (10) with reference radiation about 620 nm and a second reference light source (11) with reference radiation about 780 nm is provided, which light sources are preferably located in the excitation part (3) of the two-part measuring sleeve (2).

7. Measuring arrangement according to any of claims 1 to 6, **characterised in that** excitation filters (12a, 12b, 12c) are positioned between the light sources (4) and the luminescence-optical sensor elements (ST, SO, SG), or that the light sources (4) are embedded in a filter layer (12').

8. Measuring arrangement according to any of claims 1 to 7, **characterised in that** measuring filters (13a, 13b, 13c) are provided on the entry side of the photodetectors (6).

9. Measuring arrangement according to any of claims 1 to 8, **characterised in that** the flow-through measuring cell (1) contains at least one O₂-sensor (SO), one glucose sensor (SG) and one temperature sensor (ST).

## Revendications

1. Dispositif de mesure pour déterminer au moins un paramètre d'un échantillon sanguin comportant une cellule de mesure par passage (1) avec au moins un élément de capteur opto-luminescent (ST, SO, SG) qui peut être mis en contact avec au moins l'échantillon sanguin,
- au moins une source lumineuse (4) pour exciter l'élément de capteur opto-luminescent et au moins un photo-détecteur (6) pour recevoir le rayonnement luminescent émis par l'élément de capteur opto-luminescent,
- la source lumineuse (4) et le photo-détecteur (6) étant installés sur
des côtés opposés (7, 8) de la cellule de mesure par passage (1), dispositif **caractérisé en ce qu'**
- au moins l'élément de capteur opto-luminescent (ST, SO, SG) est installé sur le côté d'excitation (7) de la cellule de mesure à passage (1), tourné vers la source lumineuse (4),
- la source lumineuse (4) émet un rayonnement d'excitation d'une longueur d'onde inférieure à 600 nm, par exemple égale à 425 nm et le rayonnement luminescent de l'élément de capteur opto-luminescent (ST, SO, SG) se situe dans une plage de longueurs d'onde supérieures à 600 nm de sorte que le rayonnement d'excitation à travers l'échantillon sanguin est soumis à une absorption beaucoup plus forte que le rayonnement luminescent.

2. Dispositif de mesure selon la revendication 1,
**caractérisé en ce que**
du côté d'excitation (7) de la cellule de mesure par passage (1), plusieurs éléments de capteur opto-luminescents (ST, SO, SG), sont installés de préférence suivant une disposition linéaire le long de l'axe de la cellule de mesure (1'),
* à chaque élément de capteur (ST, SO, SG) est associée une source lumineuse (4) et un photo-détecteur (6) est associé au côté de mesure (8) opposé de la cellule de mesure par passage (1).

3. Dispositif de mesure selon la revendication 1 ou 2,
**caractérisé en ce que**
la cellule de mesure par passage (1) est installée de manière interchangeable dans un manchon de mesure en deux parties (2) dont la partie d'excitation (3) comporte les sources lumineuses (4), de préférence des diodes LED avec leur électronique d'excitation et leur partie de mesure (5), les photo-détecteurs (6), de préférence des photodiodes avec leur électronique de mesure.

4. Dispositif de mesure selon l'une des revendications 1 à 3,
**caractérisé en ce que**
le côté d'excitation (7) de la cellule de mesure par passage (1) comporte une couche formant miroir (9) dans la région des éléments de capteur opto-luminescents (ST, SO, SG), cette couche étant transparente pour le rayonnement d'excitation et réfléchissant le rayonnement luminescent.

5. Dispositif de mesure selon l'une des revendications 1 à 4,
**caractérisé par**
au moins une source de lumière de référence (10, 11) sur le côté d'excitation (7) de la cellule de mesure par passage (1), et dont le rayonnement de référence traverse la cellule de mesure par passage (1) et arrive sur les photo-détecteurs (6) installés sur le côté de mesure (8), opposé.

6. Dispositif de mesure selon la revendication 5,
**caractérisé par**
une première source de lumière de référence (10) avec un rayonnement de référence dans la plage de 620 nm et une seconde source de lumière de référence (11) avec un rayonnement de référence dans la plage de 780 nm, et qui se trouvent de préférence dans la partie d'excitation (3) du manchon de mesure (2) en deux parties.

7. Dispositif de mesure selon l'une des revendications 1 à 6,
**caractérisé par**
des filtres d'excitation (12a, 12b, 12c) installés entre les sources lumineuses (4) et les éléments de capteur opto-luminescents (ST, SO, SG) ou encore la source de lumière (4) intègre une couche de filtre (12').

8. Dispositif de mesure selon l'une des revendications 1 à 7,
**caractérisé par**
des filtres de mesure (13a, 13b, 13c) installés sur le côté d'entrée des photo-détecteurs (6).

9. Dispositif de mesure selon l'une des revendications 1 à 8,
**caractérisé en ce que**
la cellule de mesure par passage (1) comporte au moins un capteur d'oxygène O₂ (SO), un capteur de glucose (SG) et un capteur de température (ST).
